# EUROPEAN PATENT SPECIFICATION

(11) **EP 0 645 142 B1**
(45) Date of publication and mention of the grant of the patent: **24.07.2002**
(21) Application number: 94115306.6
(22) Date of filing: 28.09.1994
(51) Int. Cl.: A61K 35/36, A61P 7/02, A61P 37/08

(54) **A novel physiologically active substance**
Neues physiologisch aktives Mittel
Nouvelle substance physiologiquement active

(30) Priority: 28.09.1993 JP 26558993
(43) Date of publication of application: 29.03.1995
(73) Proprietor: NIPPON ZOKI PHARMACEUTICAL CO., LTD., Chuo-ku, Osaka (JP)
(72) Inventor: Konishi, Jin-emon, Musashino-shi, Tokyo (JP); Hamada, Giichi, Nishinomiya-shi, Hyogo-ken (JP)
(74) Representative: Hansen, Bernd, Dr. Dipl.-Chem.

(56) References cited:
- PATENT ABSTRACTS OF JAPAN, unexamined applications, C field, vol. 8, no. 236, Ocotber 30, 1984 THE PATENT OFFICE JAPANESE GOVERNMENT page 99 C 249; & JP-A-59 118 711 (NIHON ZOUKI SEIYAKU K.K.)
- CHEMICAL ABSTRACTS, vol. 98, no. 26, 1983, June 27, Columbus, Ohio, USA NIPPON ZOKI PAHRMACEUTICAL CO. "Analgesic and ulcer - inhibiting glycophosphopro- teins from animal tissues infected with vaccinia virus" page 383, column 1, no. 221 826z; & JP-A-58 35 117 (83 35,117)

## Description

The present invention relates to a novel physiologically active substance which is extracted from a tissue activated by internal and external stressors.

Living organisms keep each of their lives as individuals by adjusting and maintaining their physical and chemical states to and within certain stable physiological conditions corresponding to the changes in internal and external circumstances.

In order to maintain and adjust such homeostasis, the living organism always produces various substances *in vivo* and, in case of an invasion of viruses, bacteria, etc. and of a generation of tumor cells, it also produces certain substances *in vivo* which are resistant to such external and internal invasions.

However, if the above-mentioned biofunctions are unbalanced for some reason and that becomes chronic, then so-called morbidity results causing various diseases.

The ideal way of curing the disease is that the function of the organism to maintain homeostasis is activated and adjusted so that the abnormal imbalance of the disordered biofunction recovers to a normal state. It is well known that the maintenance and the normalization of the biofunction are carried out especially by various receptors on cell surfaces and the ion channels such as sodium, potassium, calcium, etc.

It is known that, upon growing older, the recovering ability of DNA against damage lowers in mammalian cells and that the production of free radicals *in vivo* promotes ageing, collagen disease and generation of cancer. Incidentally, collagen is a noncellular substance widely present in skins, blood vessels, cartilages, eye balls, kidney, etc. and, with an advance in age, a crosslinking of collageneous materials proceeds whereby their elasticity reduces and they become hard. It is well known that, in patients suffering from diabetes, an excessive crosslinking of the collageneous materials proceeds as a result of a continuing high sugar level in the blood, whereby cataracts, atherosclerosis, renal diseases, peripheral nervous disorders, etc. particularly result.

By paying attention to the function of maintaining homeostasis of living organisms which adjusts and recovers the strain in nervous, immunological and endocrine systems caused by a disorder of cell functions *in vivo* as a result of diseases and ageing, the present inventors have conducted an extensive investigation into the substances which are produced at the resisting stage of the organisms against the internal and external stresses (i.e. at the activating stage of the living tissues) and promote the natural curing ability of the organisms to participate in the normalization of the biofunctions whereby the present invention has been achieved.

As one of the mechanisms for adjusting the complicated functions *in vivo,* an enzymatic system called a kallikrein-kinin system has been known. With respect to this plasma kallikrein-kinin system, it is believed that a blood coagulation factor XII is activated due to a stimulation by lesion or invasion of the tissues *in vivo* whereby a series of the enzymatic reactions is induced. Thus, the activated blood coagulation factor XII acts on the plasma prekallikrein existing in the same plasma to convert it to a plasma kallikrein of an activated enzyme type and then the plasma kallikrein acts on the high-molecular-weight kininogen in the plasma to liberate bradykinin.

The bradykinin which is a product of the plasma kallikrein-kinin system exhibits various physiological activities such as dilation of peripheral blood vessels, acceleration of permeation of blood vessels, induction of pain, generation of inflammation, migration of leucocytes, etc. and has been known as a mediator for induction of pain, inflammation and allergic reactions. Accordingly, when an excessive liberation and production of bradykinin is inhibited, it is possible to relieve pain, inflammation, allergic syndromes, etc. and to normalize such disorders.

As mentioned above, bradykinin is liberated and produced in the reaction of the plasma kallikrein with high molecular weight kininogen and, therefore, substances which inhibit the kallikrein production in the plasma kallikrein-kinin system and prevent an excessive production of bradykinin have the possibility of being used as analgesics, antiinflammatory agents, antiallergic drugs, etc. and are highly useful as pharmaceuticals.

As a background of the present invention, following two Japanese applications filed by the same applicant of the present invention may be referred to:
1) JP-A-58 35 117 (Chemical Abstracts, Vol.98, No.221826z (1983))
2) JP-A-59 118 711 (Patent Abstracts of Japan, unexamined applications, C field, Vol.8, No.236, page 99 c 249 (1984))

Both of the applications relate to physiologically active substances obtained by the same manufacturing method which is characterized by a purification step with a ultrafiltration procedure applied to an extract from vaccinia-virus-innoculated tissues to give active substances having molecular weight less than 1,000. On the other hand, the substance of the present invention is extracted also from inflammatory tissues infected with virus, but by a different method from the above, and possess pharmacologically characteristic activities such as actions inhibiting a production of plasma kallilrein and improving peripheral circulation disturbance.

The present invention relates to a biofunction-adjusting and maintaining substance which exhibits an activity of inhibiting the production of plasma kallikrein and of improving the peripheral blood flow and recovers and normalizes the abnormal function of the diseased state.

An object of the present invention is to provide a physiologically active substance which exhibits an improving action for peripheral blood flow as well as analgesic, antiinflammatory and antiallergic actions, etc.

The present invention relates to a physiologically active substance obtainable by
A) inoculating animals or animal tissues with a stressor to activate the tissues, and
B) extracting the physiologically active substance from the activated tissue, and
   having the following additional properties:
   i) It is an amorphous, hygroscopic powder with a pale yellowish brown color containing 1 to 20 micrograms/mg of silicon, calculated as silicon.
   ii) It is soluble in water, methanol and ethanol and is insoluble in benzene and ether.
   iii) It displays a pH of from 6.0 to 8.3.
   iv) It has a peak in the ultraviolet absorption spectrum at λₘₐₓ = 265 to 275 nm.
   v) It displays the following color reactions:
      - amino acids - positive to the ninhydrin reaction.
      - sugars - positive to the orcinol-iron (III) chloride-hydrochloric acid method.
      - phosphorous - positive to the molybdenum blue method.
      - proteins - negative to the trichloroacetic acid method.
      - phenols - negative to the ferric chloride method.
   vi) It has an inhibitory action on the production of plasma kallikrein. The present invention is preferably obtained in such a manner that the activated tissues of an animal are ground down, a solvent for extraction is added, tissue residues are removed therefrom, proteins are removed, then the remainder is adsorbed with an adsorbent and the substance adsorbed therewith is eluted.
In detail the method for the production of the physiologically active substance of the invention may comprise the following steps:
   A) inoculating animals or animal tissues with a stressor to activate the tissues, and
   B) extracting the physiologically active substance from the activated tissue, wherein the extraction step (ii) is comprised of the following steps:
      a) grinding the activated tissue and adding an extracting solvent to form an emulsion,
      b) optionally subjecting the suspension to a freezing/melting treatment, an ultrasonic wave treatment, the adding of cell membrane dissolving enzymes or the adding of surface active agents,
      c) separating and removing the tissue residue,
      d) removing proteins from the extracted fraction,
      e) adjusting the extracted fraction to an acidic pH and adsorbing it with an adsorbent, and separating and collecting the adsorbent,
      f) eluting the product from the adsorbent with an extracting solvent at an alkaline pH,
      g) optionally purifying the product by chromatography, dialysis or ultrafiltration.

The present invention will be further illustrated as hereunder.

The stressor preferably is a virus or tumor cells.

The animal tissues used in the present invention are preferably cultured tissues cultured cells or inflammatory tissues of human or animal origin which are infected with virus, or chorio-allantoic membranes of embryonated eggs infected with virus. Examples of the virus used for the activation of the animal tissues as a stressor are vaccinia virus, cowpox virus, variola virus, ectromelia virus, simian pox virus and other orthopox viruses, Orf virus, paravaccinia virus, bovine nopplelike stomatitis virus and other parapoxviruses, sheep pox virus, goatpox virus, lumpy skin disease virus and other goatpox viruses, avian pox virus, hare fibroma virus and other avian pox viruses, rabbit myxoma virus, rabbit fibroma virus and other rabbit pox viruses as well as swine pox virus, Yava monkey tumor virus, Tara pox virus and other viruses belonging to the family poxvirus.
The virus preferably is a vaccinia virus. As to the tumor cells as a stressor, various tumor-cultured cell strains derived from human beings and animals may be used and anything that can be inoculated to the above animals and animal tissues will do.

With respect to the animals for preparing the activated tissues, domestic animals and fowl, such as rabbits, cows, horses, sheep, goat, swine, chickens, etc. and mammals such as monkeys, rats, mice, guinea pigs, hamsters, etc. may be used and they may be suitably selected depending upon the type of the stressors and the object.
The animal tissue is preferably rabbit skin.

With respect to the cells for the culture, any cell will do so far as the stressor used is able to grow there. Examples of such cells for the culture include various tissues (e.g. human hemocytes and placentae) and the cells of various tissues, such as kidney, skin, lung, testis, muscle, adrenal gland, thyroid gland, brain, nerve cells, hemocytes, etc. of the above-mentioned animals and embryos thereof.

Those activated tissues are aseptically collected, ground and made into an emulsified suspension by adding 1 to 5 times as much extracting solvent thereto. Examples of the extracting solvent applicable are distilled water, physiologically saline solution, weakly acidic to weakly basic buffers, etc. If necessary, stabilizers such as glycerol, antibacterial/ antiseptic agents such as phenol, inorganic salts such as sodium chloride, potassium chloride, magnesium chloride, etc. may be added thereto. At that time, the extraction can be made easier by carrying out a freezing/melting treatment, subjecting to ultrasonic waves, or adding cell membrane dissolving enzymes or surface-active agents.

The resulting milky extract is filtered or centrifuged to remove the tissue residue and then proteins are removed therefrom. Removal of the proteins can be carried out by known methods and the treatments of heating, subjecting to ultrasonic waves, adding protein denaturating agents such as acids, bases, urea, guanidine, organic solvents, surface-active agents, etc., isoolectric precipitation, salting-out, and the like. Then the proteins separated out therefrom are filtered off by means of filtration using filter paper (cellulose, nitrocellulose, etc.), glass-filter, Celite, Seitz filter, etc. as well as ultrafiltration, gel filtration, ion exchange resin, centrifugation and the like.

The resulting extracted fraction is adjusted to be acidic, preferably, to pH 3.5-5.5, by an acid such as hydrochloric acid, sulphuric acid, hydrobromic acid, etc. and adsorbed with an adsorbent. Examples of the applicable adsorbent are activated charcoal, kaolin, ion exchange resins, etc, activated charcoal being the preferred adsorbant.

The adsorbent is added to the extract followed by stirring or the extract is passed through a column filled with the adsorbent whereby the effective component can be adsorbed.
Preferably the step of adjusting the extracted fraction to an acidic pH and adsorbing it with an adsorbant, and separating and collecting the adsorbant is carried out more than once.

In eluting the substance of the present invention from the adsorbent, an extracting solvent (e.g. a basic aqueous solution, a solution in a water-miscible solvent such as alcohol or a mixed solution thereof) is added, the mixture is preferably adjusted to pH 9-12, then eluted at room temperature or by beating to some extent or with stirring and the adsorbent is removed by conventional means such as filtration, whereby elution can be achieved. Then, if necessary, means such as chromatography, ultrafiltration, dialysis using reverse osmosis filtration etc. or removal of the salt therefrom are applied whereupon the physiologically active substance of the present invention can be prepared in a purer state.

The silicon contained in the physiologically active substance of the present invention is present as water-soluble silicic acids or silicates or polymers thereof. It may be present as silicic acids such as orthosilicic acid, metasilicic acid, mesodisilicic acid, mesotrisilicic acid, mesotetrasilicic acid, etc. or alkali salts (e.g. sodium and potassium salts) thereof, in a form of monomers, or in a polymerized form. The substance of the present invention contains 1-20 micrograms/mg (preferably 1.5-15 micrograms/mg) of it when calculated as silicon.

Examples of the method for manufacturing the substance of the present invention are given below.

### [Examples]

### Example 1

The skin of a healthy adult rabbit was inoculated with vaccinia virus in order to activate it, then the activated skin was aseptically taken off, finely cut, water was added thereto and the mixture was ground using a homogenizer to prepare an emulsion. This was filtered with pressure, the resulting filtrate was adjusted to pH 5.0 with hydrochloric acid and heated at 100°C with a steam flow. Proteins were removed by filtration, the filtrate was adjusted to pH 9.1 with sodium hydroxide, heated at 100°C and filtered. The filtrate was adjusted to pH 4.1, stirred after adding 2% of activated charcoal and the mixture was filtered. To the filtrate was added 5.5% of activated charcoal and the mixture was stirred for two hours and filtered. The activated charcoal which was obtained from the first filtration was mixed with water, adjusted to pH 9.9 with sodium hydroxide, stirred at 60°C for 1.5 hours and filtered. Water was added to the first quantity of activated charcoal and the second one, adjusted to pH 10.9 with sodium hydroxide, stirred at 60°C for 1.5 hours and filtered. The filtrates were combined, neutralized with hydrochloric acid, desalted using a reverse osmotic filter membrane (molecular weight: 100) and dried in vacuo. The yield from 1 kg of the activated skin was 3 g. The physiologically active substance prepared as such exhibited the following properties.
(1) Characteristic: an amorphous and hygroscopic powder with pale yellowish brown color containing 2-10 micrograms/mg of silicon which is calculated as silicon;
(2) Solubility: it is soluble in water, methanol and ethanol and is insoluble in benzene and ether;
(3) pH; 6.0-8.3;
(4) Ultraviolet absorption: λₘₐₓ = 265-275 nm;
(5) Color reactions: amino acids (positive to the ninhydrin reaction), sugars (positive to the orcinol-iron(III) chloride-hydrochloric acid method), phosphorus (positive to the molybdenum blue method), proteins (negative to the trichloroacetic acid method) and phenols (negative to the ferric chloride method).

### Example 2.

L cells (sarcoma cells of mice) were hypodermically transplanted to C3H mice, vaccinia virus was inoculated in the same place after ten days and, five days after that, the areas of tumor inflammation were excised. The excised tissue (100 g) was finely cut, a 70% glycerol solution buffered to pH 7.0 was added, ground down using a Waring blender and an operation of freezing/melting was repeated three times. The milky ground liquid was centrifuged at 2,000 x g for one hour, the precipitates wore removed, the pH of the supernatant fluid was adjusted to 5.0, heated at 100°C and filtered. The filtrate was adjusted to pH 9.0, heated at 100°C again and filtered to remove the insoluble matter. After cooling, the filtrate was adjusted to pH 4.5, passed through a column filled with activated charcoal and the column was washed with distilled water and eluted with N/25 aqueous ammonia. Neutralization and desalting were carried out in the same manner as in Example 1 followed by drying *in vacuo* whereupon a powdery product was obtained. The physiologically-active substance of the present invention prepared as such contained a larger amount of silicic acids and the amount contained in 1 mg of the hygroscopic powder was 5-14 micrograms calculated as silicon.

The pharmacological activity of the physiologically-active substance of the present invention is as follows.

### (1) The action of inhibiting the production of plasma kallikrein.

The inhibitory action of the physiologically-active substance against the production of plasma kallikrein was measured according to a method mentioned in the literature [*Kiso to Rinsho,* vol.20. no.17, pages 399-405(1986)].

Thus, a suspension of kaolin was added to the normal human plasma diluted with a physiologically saline solution, a lima bean trypsin inhibitor was added thereto after certain period to stop the reaction of the kallikrein production and then the resulting kallikrein was determined by a synthetic substrate (D-Pro-Phe-Arg-p-nitroaniline). The substance to be tested was made copresent in the above system whereby the inhibitory action of the substance to be tested against the production of kallikrein was determined.

One example of the result is given in Table 1. The potency of the activity is given in terms of a concentration (IC₅₀) whereby the production of the plasma kallikrein was inhibited to an extent of 50%.

**Table 1.**

| Substance Tested | IC₅₀ (micrograms/ml) |
|---|---|
| The substance of this invention | 35 |
| Indomethacin | 370 |
| Ketoprofen | 400 |
| Ibuprofen | 700 |
| Pentazocine | 1,600 |

### (2) The action of improving the peripheral circulation disturbance.

As an index for the action of the substance of the present invention for improving the abnormal sensation, the action of improving the peripheral blood circulation disturbance by chinoform was measured. Thus, chinoform was intraperitoneally administered to rats for 27 days with gradually increasing doses to cause a peripheral circulation disturbance, then the hind paws were dipped in water of 5°C for two minutes to give a low temperature load and the progress in the recovery of the paw temperature was subjected to a picture analysis by means of a thermography to evaluate the improving action against the peripheral circulation disturbance. The substance of the present invention was intraveously administered for a continuing seven days from the 21st day after the administration of chinoform.

An example of the results to given in Table 2.

**Table 2.**

| Average Skin Temp of the Hind Paws (°C)15 Minutes after Releasing the Low Temp Load | |
|---|---|
| Non-treated group | 27.3 ± 0.8 |
| Control group (treated with chinoform) | 24.1 ± 0.3 |
| Groups treated with the substance of the invention | |
| 50 mg/kg | 25.7 ± 0.3 |
| 100 mg/kg | 28.2 ± 0.7 |

It is clear from the result of Table 1 that the physiologically active substance of the present invention exhibits an extremely excellent inhibitory action against the production of plasma kallikrein. As mentioned already, the plasma kallikrein acts on a high-molecular-weight kininogen whereby bradykinin is liberated and produced. Said bradykinin is known as a mediator for inducing peripheral blood vessel dilation, pain, inflammation and allergic reactions. Thus, when production of the plasma kallikrein is inhibited, the liberation of bradykinin can be inhibited and, accordingly, the substance of the present invention exhibiting an excellent inhibitory action against the plasma kallikrein production is very highly useful as a pharmaceutical such as, for example, an analgesic, antiinflammatory and antiallergic drug. Further, the pharmacological activity of the physiologically-active substance of the present invention was tested in various systems both *in vitro* and *in vivo* whereupon it was found that the substance of the present invention exhibits excellent pharmacological activities such as a peripheral blood flow improving action, and antiinflamatory and antiallergic actions.

The physiologically active substance of the present invention can be made into various pharmaceutical preparations by a suitable combination with various pharmaceutical carriers or diluents by conventional means whereby solid, semisolid, liquid or aerosol preparations for oral or parenteral use are obtained in the formulation, the substance of the present invention may be used solely or together with other pharmaceutically active components.

In the case of injections, a solution or a suspension may be prepared using an aqueous or nonaqueous solvent such as distilled water for injections, physiologically saline solution. Ringer's solution, plant oil, synthetic fatty acid glycerides, higher fatty acid esters, propylene glycol, etc. followed by adjusting the pH and isotonization.

In the case of preparations for oral administration, the substance of the invention *per se* or its mixture with suitable additives such as fillers (e.g. lactose, mannitol, corn starch, crystalline cellulose, etc.) may be combined together with binders (e.g. gum arabicum, corn starch, gelatin, etc.), disintegrating agents (e.g. corn starch, potato starch, carmerose, carmerose calcium, etc.), lubricants (e.g. talc, magnesium stearate, etc.), bulking agents, moisturizers, buffers, preservatives, perfumes and the like to give tablets, diluted powders, granules or capsules. Depending upon the state of the patient and on the type of the disease, the preparation forms which are other than the above-given ones and are suitable as a therapy therefore such as suppositories, inhalating agents, aerosols, ointments, cataplasms, eye drops, etc. may be prepared.

## Claims

1. A physiologically active substance obtainable by extracting the physiologically active substance from activated tissue of inoculated animals or animal tissues with a stressor to activate the tissues, and
having the following additional properties:
i) It is an amorphous, hygroscopic powder with a pale yellowish brown colour containing 1 to 20 micrograms/mg of silicon, calculated as silicon.
ii) It is soluble in water, methanol and ethanol and is insoluble in benzene and ether.
iii) It displays a pH of from 6.0 to 8.3.
iv) It has a peak in the ultraviolet absorption spectrum at λmax = 265 to 275 nm.
v) It displays the following colour reactions:
• Amino acids - positive to the ninhydrin reaction.
• Sugars - positive to the orcinol-iron (III) chloride-hydrochloric acid method.
• Phosphorous - positive to the molybdenum blue method.
• Proteins - negative to the trichloroacetic acid method.
• Phenols - negative to the ferric chloride method.
vi) It has an inhibitory action on the production of plasma kallikrein.

2. A physiologically active substance as defined in claim 1 wherein said at least one silicon component comprises at least one member selected from the group consisting of water-soluble silicic acids, water soluble silicates, polymers of water-soluble silicic acids, and polymers of water-soluble silicates.

3. A physiologically active substance as defined in claim 1 or 2 containing 2 to 10 micrograms/mg of silicon, calculated as silicon.

4. A physiologically active substance as defined in claim 1 or 2 containing 5 to 14 micrograms/mg of silicon, calculated as silicon.

5. A pharmaceutical composition comprising a physiologically active substance as defined in any one of claims 1 to 4 and a pharmaceutically acceptable carrier.

6. The use of a physiologically active substance as defined in any one of claims 1 to 4 in the manufacture of a medicament effective as a peripheral blood flow improving agent, an analgesic agent, an anti-inflammatory agent, an antiallergic agent, or a plasma kallikrein production inhibiting agent.

7. The use according to claim 6 in the manufacture of a peripheral blood flow improving agent.

8. The use according to claim 6 in the manufacture of an analgesic agent.

9. The use according to claim 6 in the manufacture of an anti-inflammatory agent.

10. The use according to claim 6 in the manufacture of an anti-allergic agent.

11. The use according to claim 6 in the manufacture of a plasma kallikrein production inhibiting agent.

## Patentansprüche

1. Eine physiologisch aktive Substanz, die durch das Extrahieren der physiologisch aktiven Substanz aus aktiviertem Gewebe erhältlich ist, das aus Tieren oder tierischen Geweben stammt, welche man mit einer streßauslösenden Substanz zur Aktivierung der Gewebe geimpft hat, und welche die folgenden zusätzlichen Eigenschaften aufweist:
i) sie ist ein amorphes, hygroskopisches Pulver, mit einer schwach gelblich braunen Farbe, welches 1 bis 20 µg/mg Silicium, berechnet als Silicium, enthält;
ii) sie ist löslich in Wasser, Methanol und Ethanol und unlöslich in Benzol und Ether;
iii) sie weist einen pH von 6,0 bis 8,3 auf;
iv) sie hat einen Peak im UV-Absorptionsspektrum bei λmax = 265 bis 275 nm;
v) sie zeigt die folgenden Farbreaktionen:
• Aminosäuren - positiv auf die Ninhydrin-Reaktion;
• Zucker - positiv auf das Orcinol-Eisen(III)-chlorid-Salzsäure-Verfahren;
• Phosphor - positiv auf das Molybdänblau-Verfahren;
• Proteine - negativ auf das Trichloressigsäure-Verfahren;
• Phenole - negativ auf das Eisen(III)-chlorid-Verfahren;
vi) sie zeigt eine Inhibitorwirkung für die Herstellung von Plasmakallikrein.

2. Physiologisch aktive Substanz gemäß Anspruch 1, worin die zumindest eine Silicium-Komponente mindestens eine Verbindung ausgewählt aus wasserlöslichen Kieselsäuren, wasserlöslichen Silikaten, Polymeren von wasserlöslichen Kieselsäuren und Polymeren von wasserlöslichen Silikaten ist.

3. Physiologisch aktive Substanz gemäß Anspruch 1 oder 2, welche 2 bis 10 µg/mg Silicium, berechnet als Silicium enthält.

4. Physiologisch aktive Substanz gemäß Anspruch 1 oder 2, die 5 bis 14 µg/mg Silicium, berechnet als Silicium enthält.

5. Pharmazeutische Zusammensetzung, die eine physiologisch aktive Substanz, wie sie in irgendeinem der Ansprüche 1 bis 4 definiert ist und einen pharmazeutisch annehmbaren Träger enthält.

6. Verwendung der physiologisch aktiven Substanz wie sie in irgendeinem der Ansprüche 1 bis 4 definiert ist, bei der Herstellung eines Medikaments, das als Mittel zur Verbesserung des peripherischen Blutflusses, als schmerzlinderndes Mittel, als entzündungshemmendes Mittel, als antiallergisches Mittel, oder als Inhibitor für die Plasmakallikrein-Herstellung wirksam ist.

7. Verwendung gemäß Anspruch 6 bei der Herstellung eines den peripherischen Blutfluß verbessernden Mittels.

8. Verwendung gemäß Anspruch 6 bei der Herstellung eines schmerzlindernden Mittels.

9. Verwendung gemäß Anspruch 6 bei der Herstellung eines entzündungshemmenden Mittels.

10. Verwendung gemäß Anspruch 6 bei der Herstellung eines antiallergischen Mittels.

11. Verwendung gemäß Anspruch 6 bei der Herstellung eines die Plasmakallikrein-Herstellung inhbierenden Mittels.

## Revendications

1. Substance physiologiquement active pouvant être obtenue par extraction de cette substance physiologiquement active à partir de tissu activé d'animaux inoculés ou de tissus d'animaux exposés à un agent stressant pour activer lesdits tissus, et
présentant les propriétés complémentaires suivantes :
i) il s'agit d'une poudre hygroscopique, amorphe de couleur brune jaunâtre pâle contenant de 1 à 20 µg/mg de silicium, exprimés en terme de silicium.
ii) elle est soluble dans l'eau, le méthanol et l'éthanol mais insoluble dans le benzène et l'éther.
iii) elle présente un pH compris entre 6,0 et 8,3.
iv) elle présente un pic d'absorption dans le domaine ultraviolet à λmax = 265 à 275 nm.
v) elle présente les réactions colorées suivantes :
• Acides aminés : réaction positive à la ninhydrine
• Glucides : réaction positive par la technique d'orcinol-chlorure de fer (III) - acide chlorhydrique.
• Phosphore - réaction positive par la technique de bleu de molybdène
• Protéines - réaction négative par la technique d'acide trichloroacétique.
• Dérivés phénoliques - réaction négative par la technique de chlorure ferrique.
vi) possède une action inhibitrice sur la production de kallikréine plasmatique.

2. Substance physiologiquement active telle que définie dans la revendication 1, dans laquelle ledit au moins composant silicique comprend au moins un membre choisi dans le groupe consistant en les acides siliciques solubles dans l'eau, les silicates solubles dans l'eau, des polymères d'acides siliciques solubles dans l'eau, et des polymères de silicates solubles dans l'eau.

3. Substance physiologiquement active telle que définie dans la revendication 1 ou 2 contenant de 2 à 10 µg/mg de silicium, exprimés en terme de silicium.

4. Substance physiologiquement active telle que définie dans la revendication 1 ou 2, contenant de 5 à 14 µg/mg de silicium, exprimés en terme de silicium.

5. Composition pharmaceutique comprenant une substance physiologiquement active telle que définie dans l'une quelconque des revendications 1 à 4 et un véhicule pharmaceutiquement acceptable.

6. Utilisation d'une substance physiologiquement active telle que définie dans l'une quelconque des revendications 1 à 4 dans l'obtention d'un médicament efficace en tant qu'agent activateur de la circulation sanguine périphérique, agent antalgique, agent anti-inflammatoire, agent antiallergique ou agent inhibiteur de la production de kallikréine plasmatique.

7. Utilisation selon la revendication 6 dans l'obtention d'un agent activateur de la circulation sanguine périphérique.

8. Utilisation selon la revendication 6 dans l'obtention d'un agent antalgique.

9. Utilisation selon la revendication 6 dans l'obtention d'un agent anti-inflammatoire.

10. Utilisation selon la revendication 6 dans l'obtention d'un agent antiallergique.

11. Utilisation selon la revendication 6 dans l'obtention d'un agent inhibiteur de la production de kallikréine plasmatique.
